# EUROPEAN PATENT APPLICATION

(11) **EP 4 691 457 A1**
(43) Date of publication of application: **11.02.2026**
(21) Application number: 24781337.1
(22) Date of filing: 01.04.2024
(51) Int. Cl.: A61K 9/00, A61K 47/14, A61K 47/26, A61K 9/12, A61K 31/506, A61P 17/14

(54) **COMPOSITION CONTAINING MINOXIDIL FOR TREATING HAIR LOSS**

(30) Priority: 30.03.2023 KR 20230042380
(71) Applicant: Sinsin Pharm Co., Ltd., Sejong 30002 (KR)
(72) Inventor: KIM, Geon Woo, Seoul 02873 (KR); HAN, Mun Seok, Suwon-si Gyeonggi-do 16325 (KR); LEE, Woo Young, Yongin-si Gyeonggi-do 16856 (KR); KIM, Sang Lin, Seoul 01901 (KR); LEE, Byoung Ki, Seoul 05280 (KR)
(74) Representative: Louis Pöhlau Lohrentz
(86) International application number: PCT/KR2024/004198
(87) International publication number: WO 2024/205347

(57) **Abstract**

The present application relates to a composition containing minoxidil or a pharmaceutically acceptable salt thereof for treating hair loss. The composition includes a lipophilic transdermal absorption enhancer, which can improve the skin permeability of minoxidil.

## Description

### TECHNICAL FIELD

The present invention relates to a composition containing minoxidil for treating hair loss.

### BACKGROUND ART

Hair loss treatments are primarily being developed as oral formulations containing finasteride or dutasteride as the main ingredient or liquid formulations containing minoxidil as the main ingredient. Liquid formulations are solutions containing transdermal absorption enhancers, antioxidants, surfactants, humectants, and pH regulators dissolved in water and/or ethanol to increase the stability and skin permeability of the active ingredient.

Liquid formulations containing minoxidil have the disadvantage of causing the medication to run after application onto the application region. Therefore, a foam aerosol type product, represented by the brand name Rogaine^{®} (Johnson & Johnson Consumer Inc.; 5% minoxidil foam aerosol), has been developed and is currently available on the market. This medication, which forms a foam when sprayed as an aerosol, uses cetanol to promote skin permeation. Cetanol reacts with lactic acid, a pH regulator, to form cetyl lactate, thereby enhancing skin absorption and permeation.

### DETAILED DESCRIPTION OF THE INVENTION

### TECHNICAL PROBLEM

The present invention provides a hair loss treatment composition with improved minoxidil skin permeability.

### TECHNICAL SOLUTION

The present invention relates to a pharmaceutical composition including minoxidil or a pharmaceutically acceptable salt thereof; and a lipophilic transdermal absorption enhancer. The composition of the present invention may further include a surfactant. The pharmaceutical composition according to the present invention may be used for the treatment of hair loss in the form of a foam aerosol formulation.

The composition of the present invention may further include as a solvent one or more selected from the group consisting of water and ethanol. Preferably, volatile ethanol may be used as a solvent, and water may optionally be included (e.g., less than 70% by weight based on the total weight of the composition).

In the composition of the present invention, the lipophilic transdermal absorption enhancer is preferably a propylene glycol fatty acid ester. In the present invention, the propylene glycol fatty acid ester may preferably be propylene glycol monocaprylate, propylene glycol dicaprylate/dicaprate, propylene glycol monolaurate, propylene glycol monooleate, propylene glycol monostearate, or the like. Most preferably, propylene glycol monocaprylate is used.

In the present invention, the transdermal absorption enhancer may be included in an amount of 0.01% to 15% by weight, preferably 0.1% to 12% by weight, based on the total weight of the composition.

In the present invention, a preferred surfactant is a sorbitan ester (e.g., sorbitan stearate), a polysorbate (polyoxyethylene sorbitan fatty acid ester), or a polyoxyethylene alkyl ether, and may be included in an amount of 0.01% to 10% by weight, preferably 0.1% to 7% by weight, based on the total weight of the composition.

In addition, the composition of the present invention may further include one or more of a humectant, a pH regulator, and an antioxidant.

Any commonly used humectant (e.g., glycerin, sodium hyaluronate, mannitol, xylitol, etc.) may be used as a humectant in the present invention, but glycerin is preferably used. The humectant may be included in an amount of 0.1% to 5% by weight based on the total weight of the composition of the present invention.

Any commonly used pH regulator may be used as a pH regulator in the present invention, and an organic acid such as citric acid or lactic acid is preferably used. The pH regulator may be included in an amount of 0.1% to 5% by weight based on the total weight of the composition of the present invention.

Any commonly used antioxidant may be used as an antioxidant in the present invention, and butylated hydroxytoluene (BHT) is preferably used. The antioxidant may be included in an amount of 0.1% to 5% by weight based on the total weight of the composition of the present invention.

The pharmaceutical composition according to the present invention may be used for the treatment of hair loss and may be formulated as a liquid preparation or a foam aerosol preparation.

### ADVANTAGEOUS EFFECTS

The pharmaceutical composition according to the present invention has excellent skin permeability.

### DESCRIPTION OF THE DRAWINGS

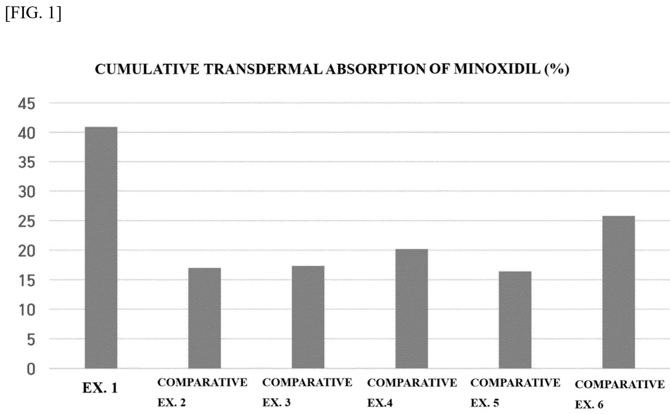

FIG. 1 is a graph showing cumulative skin permeation amount per unit area over 24 hours when a composition according to the present invention is applied.

### MODES OF THE INVENTION

Hereinafter, the present invention will be described in detail based on examples to facilitate understanding of the present invention. However, the following examples are intended only to illustrate the content of the present invention and are not intended to limit the spirit or scope of the present invention in any way. These examples are provided to more fully describe the present invention to those of ordinary skill in the art.

### Example 1: Preparation of compositions

The compositions of Example 1 and Comparative Examples 3 to 6 were prepared according to the ingredients and amounts shown in Table 1 below.

**[Table 1]**

| Classification | Ingredients | Example 1 | Comparative Example 3 | Comparative Example 4 | Comparative Example 5 | Comparative Example 6 |
|---|---|---|---|---|---|---|
| Main ingredient | Minoxidil | 4.75% | 4.75% | 4.75% | 4.75% | 4.75% |
| Solvent | Ethanol | 30.00% | 30.00% | 30.00% | 30.00% | 30.00% |
| | Propylene glycol monocaprylate | 10.00% | - | - | - | - |
| Transdermal absorption enhancer | Propylene glycol monolaurate | - | 10.00% | - | - | - |
| | Propylene glycol | - | - | 10.00% | - | - |
| | Glyceryl monooleate | - | - | - | 10.00% | - |
| | Sorbitan monooleate | - | - | - | - | 10.00% |
| Surfactant | Sorbitan stearate | 2.00% | 2.00% | 2.00% | 2.00% | 2.00% |
| | Polysorbate 60 | 2.00% | 2.00% | 2.00% | 2.00% | 2.00% |
| Antioxidant | Butylated hydroxytoluene | 0.10% | 0.10% | 0.10% | 0.10% | 0.10% |
| pH regulator | Citric acid | 0.80% | 0.80% | 0.80% | 0.80% | 0.80% |
| | Lactic acid | 2.00% | 2.00% | 2.00% | 2.00% | 2.00% |
| Emulsifier | Polyoxyethylene lauryl ether | 2.00% | 2.00% | 2.00% | 2.00% | 2.00% |
| Humectant | Glycerin | 2.00% | 2.00% | 2.00% | 2.00% | 2.00% |
| Purified water | | 39.35% | 39.35% | 39.35% | 39.35% | 39.35% |
| Liquefied petroleum gas (LPG) | | 5.00% | 5.00% | 5.00% | 5.00% | 5.00% |
| Total | | 100.00% | 100.00% | 100.00% | 100.00% | 100.00% |

Specifically, among the above ingredients, an emulsion was prepared by mixing the ingredients except for the liquefied petroleum gas (LPG) ingredient, which is a propellant, and after filling this emulsion into a foam aerosol container, the propellant LPG was pressurized and filled into the container to prepare a foam aerosol formulation.

### Experimental Example 1: Flowability evaluation

The flowability of Example 1 and a commercially available liquid formulation containing minoxidil (Comparative Example 2) was evaluated using the method described below.

A polyethylene terephthalate (PET) film with a silica coating on one side surface for release properties was placed on an inclined surface with an inclination of 10° to construct an inclined channel for flowability evaluation. 1 g of the compositions of Example 1 and Comparative Example 2 were each applied to the top of the inclined channel, and the time required for the compositions to flow 5 cm was measured. Flowability evaluation was performed five times for each composition, and the results are shown in Table 2.

**[Table 2]**

| Classification | Example 1 | Comparative Example 2 |
|---|---|---|
| Session 1 | Not flowing | 26 seconds |
| Session 2 | Not flowing | 26 seconds |
| Session 3 | Not flowing | 25 seconds |
| Session 4 | Not flowing | 26 seconds |
| Session 5 | Not flowing | 27 seconds |
| Average | - | 26 seconds |
| Flowability (cm/s) | - | 0.19 cm/s |

As can be seen from the results in Table 2 above, Example 1 does not flow on an inclined surface, thereby improving convenience in use compared to Comparative Example 2. In other words, when the composition according to the present invention is applied to the hair on the scalp, the medicinal composition does not flow, thereby allowing the medicinal effect to be continuously exerted and also ensuring that daily life is not disrupted after application.

### Experimental Example 2: Evaluation of artificial skin permeability

The compositions of Example 1 and Comparative Examples 3 to 6, as well as a currently commercially available topical hair loss treatment containing minoxidil (Comparative Example 2), were used to evaluate skin permeability using the method described below.

A Strat-M^{™} membrane (transdermal diffusion test membrane, Milipore) was used as an artificial skin, and 1 mL of each of the compositions of Example 1 and Comparative Examples 2 to 6 was applied to each donor compartment. Skin permeability tests were performed three times (n=3), and the skin permeability test conditions using a Franz diffusion cell are described below.
- Diffusion area: 1.767 cm²
- Sample loading: 1 mL each of the compositions of Example 1 and Comparative Examples 2 to 6
- Receiver compartment: 7 mL of phosphate buffered saline (PBS) (pH 7.4)
- Stirring speed: 600 rpm
- Temperature: 32 °C
- Sample collection: 200 µl of the sample was collected from each receiver compartment at 1 hour, 2 hours, 4 hours, 6 hours, 12 hours, 18 hours, and 24 hours, and then 200 µl of the PBS was supplemented.

The amount of minoxidil present in the collected samples was analyzed using high-performance liquid chromatography (HPLC). HPLC analysis was performed under the following conditions:

### <HPLC Conditions>

- Column: Hypersil Gold C18 (250 x 4.6 mm, 5 µm)
- Column temperature: 40 °C
- Mobile phase: Methanol: water: acetic acid = 700:300:10 (Docusate sodium 3.0 g/L, perchloric acid → pH 3.0)
- Flow rate: 1.0 mL/min
- Detection: 254 nm
- Sample injection volume: 10 µl

The analysis results are shown in Table 3 and FIG. 1.

**[Table 3]**

| Classification | 24 hours |
|---|---|
| | Permeation amount per unit area (%) |
| Example 1 | 40.92 |
| Comparative Example 2 | 16.98 |
| Comparative Example 3 | 17.32 |
| Comparative Example 4 | 20.18 |
| Comparative Example 5 | 16.42 |
| Comparative Example 6 | 25.78 |

As can be seen from the results in Table 3 above, Example 1 according to the present invention exhibited a significantly higher skin permeation concentration than the comparative examples.

These results indicate that propylene glycol monocaprylate exhibits significantly superior skin permeation enhancing effects compared to other transdermal absorption enhancers (propylene glycol, glyceryl monooleate, and sorbitan monooleate).

The above experiments indicate that the minoxidil-containing formulation according to the present invention ensured use convenience by preventing flow on an inclined surface and exhibited improved minoxidil transdermal permeation.

### INDUSTRIAL APPLICABILITY

The minoxidil-containing composition according to the present invention has excellent skin permeability and can be effectively used as a hair loss treatment.

## Claims

1. A pharmaceutical composition comprising: minoxidil or a pharmaceutically acceptable salt thereof; and
a lipophilic transdermal absorption enhancer.

2. The pharmaceutical composition according to claim 1, wherein the lipophilic transdermal absorption enhancer is a propylene glycol fatty acid ester.

3. The pharmaceutical composition according to claim 2, wherein the propylene glycol fatty acid ester is one or more selected from the group consisting of propylene glycol monocaprylate, propylene glycol dicaprylate/dicaprate, propylene glycol monolaurate, propylene glycol monooleate, and propylene glycol monostearate.

4. The pharmaceutical composition according to claim 2, wherein the propylene glycol fatty acid ester is one or more selected from the group consisting of propylene glycol monocaprylate and propylene glycol monolaurate.

5. The pharmaceutical composition according to claim 1 or 2, further comprising a surfactant.

6. The pharmaceutical composition according to claim 5, wherein the surfactant is one or more selected from the group consisting of a sorbitan ester, a polysorbate, and a polyoxyethylene alkyl ether.

7. The pharmaceutical composition according to claim 5, wherein the surfactant is one or more selected from the group consisting of a sorbitan stearate, a polysorbate 60, and a polyoxyethylene lauryl ether.

8. The pharmaceutical composition according to claim 1, wherein the solvent is water, ethanol, or a mixture of water and ethanol.

9. The pharmaceutical composition according to claim 1, further comprising one or more of a surfactant, a humectant, a pH regulator, and an antioxidant.

10. The pharmaceutical composition according to claim 1 or 2, wherein the pharmaceutical composition is a liquid formulation for treating hair loss.

11. The pharmaceutical composition according to claim 1 or 2, wherein the pharmaceutical composition is a foam aerosol formulation for treating hair loss.
